# EUROPEAN PATENT APPLICATION

(11) **EP 4 825 958 A1**
(43) Date of publication of application: **21.10.2026**
(21) Application number: 25738814.0
(22) Date of filing: 07.01.2025
(51) Int. Cl.: C12N 1/20, A01N 63/22, A01P 21/00, C12R 1/07

(54) **NOVEL BACILLUS AMYLOLIQUEFACIENS STRAIN HAVING ACTIVITY OF PROMOTING PLANT RESISTANCE AGAINST DROUGHT STRESS AND USES THEREOF**

(30) Priority: 08.01.2024 KR 20240003017
(71) Applicant: CJ Cheiljedang Corporation, Seoul 04560 (KR)
(72) Inventor: OH, Young Joo, Seoul 04560 (KR); KANG, Yu Na, Seoul 04560 (KR); LEE, Mi Rong, Seoul 04560 (KR); SON, Ju Yeon, Seoul 04560 (KR); YUN, Yeo Hong, Seoul 04560 (KR)
(74) Representative: Meissner Bolte Partnerschaft mbB
(86) International application number: PCT/KR2025/000332
(87) International publication number: WO 2025/150846

(57) **Abstract**

The present disclosure relates to a novel *Bacillus amyloliquefaciens* strain that enhances the stress resistance of plants and promotes the growth of plants. The *Bacillus amyloliquefaciens* N474 CP02-1004 strain, the *Bacillus amyloliquefaciens* N481 CP02-1005 strain, or a culture thereof of the present disclosure can effectively mitigate the reduction in fresh weight of plants and water amount in plants caused by drought stress.

## Description

### Technical Field

### [Cross reference to related application]

The present disclosure claims the benefit of priority based on Korean Patent Application No. 10-2024-0003017 filed on January 8, 2024, and the entire contents of which are incorporated as part of the present specification.

The present disclosure relates to a novel *Bacillus amyloliquefaciens* strain for enhancing drought stress resistance in plants and the use thereof.

### Background Art

Due to climate change, crops are exposed to various unpredictable environmental stresses, leading to significant issues with declining productivity. In particular, due to abnormal climate conditions caused by global warming, the effects of heat waves, droughts, and the expansion of subtropical deserts are significantly impacting crop production worldwide every year. Plants have a significantly less ability to avoid various environmental stresses encountered during their survival process compared to animals, and therefore, environmental stresses such as low temperature or high temperature, salinity, or dryness have a significant impact on plant survival, growth, development, and yield. Among these, drought stress is the biggest issue worldwide, and the reduction in crop yields caused by drought stress is a challenge that must be addressed.

Water is an essential element for plant growth and has a significant impact on overall plant growth and development processes, such as photosynthesis, respiration, and nutrient absorption and transport, thereby being closely related to crop productivity.

Meanwhile, with recent rising social concern over the adverse effects of agrochemicals, there is an increasing demand for environmentally friendly products that can replace chemical fertilizers and pesticides. Therefore, it is necessary to develop environmentally friendly products that can promote plant production by enhancing drought stress resistance in plants.

Korean Patent No. 10-2239389 provides a *Bacillus butanolivorans* strain having the activity of reducing drought stress of plants or enhancing the growth of plants, and Korean Patent No. 10-2324981 provides *Pseudomonas Fluorescens* strain inducing resistance to drought stress of plants.

### [Prior Art Documents]

### [Patent Documents]

Korean Laid-open Patent Application No. 2023-0109295
Korean Patent No. 10-2239389
Korean Patent No. 10-2324981

### Disclosure

### Technical Problem

As a result of intensive research to discover a novel strain for enhancing plant stress resistance and promoting plant growth, the present inventors have experimentally demonstrated that the strains of the present disclosure have an activity of increasing fresh weight and water content reduced under drought stress conditions, thereby completing the present disclosure.

Therefore, it is an object of the present disclosure to provide a novel strain that enhances plant stress resistance and promotes plant growth.

It is another object of the present disclosure to provide a composition for enhancing plant stress resistance and promoting plant growth.

It is another object of the present disclosure to provide a method for enhancing plant stress resistance or a method for promoting plant growth.

### Technical Solution

Each description and embodiment disclosed in the present disclosure may also be applied to each different description and embodiment. That is, all combinations of the various elements disclosed in the present disclosure fall within the scope of the present disclosure. In addition, it cannot be seen that the scope of the present disclosure is limited by the specific descriptions described below. In addition, a person skilled in the art can recognize or ascertain, using only routine experiment, numerous equivalents to the specific embodiments of the present disclosure described in the present disclosure. In addition, such equivalents are intended to be included in the present disclosure.

In order to achieve the above objects, one aspect of the present disclosure provides a *Bacillus amyloliquefaciens* N474 CP02-1004 strain deposited under Accession No. KCCM13393P.

Another aspect of the present disclosure provides a *Bacillus amyloliquefaciens* N481 CP02-1005 strain deposited under Accession No. KCCM13394P.

Another aspect of the present disclosure provides a composition for enhancing plant stress resistance, comprising one or more selected from the group consisting of the *Bacillus amyloliquefaciens* N474 CP02-1004 strain deposited under Accession No. KCCM13393P or the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

Another aspect of the present disclosure provides a composition for promoting plant growth, comprising one or more selected from the group consisting of the *Bacillus amyloliquefaciens* N474 CP02-1004 strain deposited under Accession No. KCCM13393P or the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

Another aspect of the present disclosure provides a method for enhancing plant stress resistance, comprising treating a plant, an area surrounding the plant, or both, with one or more selected from the group consisting of the *Bacillus amyloliquefaciens* N474 CP02-1004 strain deposited under Accession No. KCCM13393P or the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

Another aspect of the present disclosure provides a method for promoting plant growth, comprising treating a plant, an area surrounding the plant, or both, with one or more selected from the group consisting of the *Bacillus amyloliquefaciens* N474 CP02-1004 strain deposited under Accession No. KCCM13393P or the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

Hereinafter, the present disclosure will be described in detail.

### 1. Novel strains and activity thereof

One aspect of the present disclosure provides a *Bacillus amyloliquefaciens* N474 CP02-1004 strain deposited under Accession No. KCCM13393P.

In addition, another aspect of the present disclosure provides a *Bacillus amyloliquefaciens* N481 CP02-1005 strain deposited under Accession No. KCCM13394P.

The *Bacillus amyloliquefaciens* N474 CP02-1004 strain deposited under Accession No. KCCM13393P was deposited internationally with the Korean Culture Center of Microorganisms, which is an international depository institution under the Budapest Treaty, and was assigned the Accession No. KCCM13393P on September 21, 2023.

In the present disclosure, the *Bacillus amyloliquefaciens* strain deposited under Accession No. KCCM13393P is designated as the N474 CP02-1004 strain, and accordingly, may be used interchangeably with the terms "*Bacillus amyloliquefaciens* N474 CP02-1004 microorganism", "*Bacillus amyloliquefaciens* N474 CP02-1004 strain", "*Bacillus amyloliquefaciens* N474 strain", "N474 CP02-1004 strain", "N474 microorganism", and "N474 strain" throughout this specification. The 16s rRNA gene nucleotide sequences of the N474 strain are set forth in SEQ ID NO: 1.

The *Bacillus amyloliquefaciens* N474 CP02-1004 strain deposited under Accession No. KCCM13393P may comprise a 16S rRNA gene nucleotide sequence having about 95% or more, about 96% or more, about 97% or more, about 98% or more, about 99% or more, about 99.5% or more, or about 99.9% or more sequence identity to the nucleotide sequence of SEQ ID NO: 1. Specifically, the N474 CP02-1004 strain may comprise a 16S rRNA gene nucleotide sequence containing the nucleotide sequence of SEQ ID NO: 1.

The *Bacillus amyloliquefaciens* N481 CP02-1005 strain deposited under Accession No. KCCM13394P was deposited internationally with the Korean Culture Center of Microorganisms, which is an international depository institution under the Budapest Treaty, and was assigned the Accession No. KCCM13394P on September 21, 2023.

In the present disclosure, the *Bacillus amyloliquefaciens* strain deposited under Accession No. KCCM13394P is designated as the N481 CP02-1005 strain, and accordingly, may be used interchangeably with the terms "*Bacillus amyloliquefaciens* N481 CP02-1005 microorganism", "*Bacillus amyloliquefaciens* N481 CP02-1005 strain", "*Bacillus amyloliquefaciens* N481 strain", "N481 CP02-1005 strain", "N481 microorganism", and "N481 strain" throughout this specification. The 16s rRNA gene nucleotide sequences of the N481 strain are set forth in SEQ ID NO: 2.

The *Bacillus amyloliquefaciens* N481 CP02-1005 strain deposited under Accession No. KCCM13394P may comprise a 16S rRNA gene nucleotide sequence having about 95% or more, about 96% or more, about 97% or more, about 98% or more, about 99% or more, about 99.5% or more, or about 99.9% or more sequence identity to the nucleotide sequence of SEQ ID NO: 2. Specifically, the N481 CP02-1005 strain may comprise a 16S rRNA gene nucleotide sequence containing the nucleotide sequence of SEQ ID NO: 2.

In one embodiment, the N474 CP02-1004 strain and/or the N481 CP02-1005 strain may enhance plant stress resistance and/or promote plant growth.

In one embodiment, the plant stress may be drought stress.

In one embodiment, the drought stress encompasses water loss due to drought, a reduction in plant fresh weight, a reduction in crop yield, inhibition of normal plant growth, and vulnerability to pests and diseases.

In one embodiment, the "enhancing plant stress resistance" may be reducing drought stress.

The term "reducing drought stress" in this specification may refer to, but is not limited to, restoring the plant water content reduced by drought stress, preserving fresh weight, preserving crop yield, promoting growth, and imparting resistance to pests and diseases.

In the present disclosure, the term "plant growth" refers to the process in which plant seeds germinate to develop roots, stems, leaves, and flowers, or subsequently and gradually increase in size, weight, and quantity, and the term "promoting plant growth" refers to promoting the germination of plant seeds or an increase in the size and/or weight of germinated plants.

In the present disclosure, the "plant" includes organisms belonging to the Kingdom Plantae that perform photosynthesis, and is not taxonomically limited to any specific plant.

In the present disclosure, the "plant" may be one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

The "Cucurbitaceae" plants may include, for example, cucumber, oriental melon, watermelon, cantaloupe, honeydew, melon, pumpkin, zucchini, and squash, and specifically, the Cucurbitaceae plants may be cucumber, oriental melon, watermelon, or melon, but are not limited thereto.

The "Brassicaceae" plants may include, for example, Chinese cabbage, arugula, broccoli, bok choy, Brussels sprouts, cabbage, cauliflower, canola, collard greens, daikon, garden cress, horseradish, kale, mustard, radish, rapeseed, rutabaga, turnip, wasabi, watercress, and *Arabidopsis thaliana*, and specifically, the Brassicaceae plants may be, but are not limited to, Chinese cabbage, radish, mustard, rapeseed, or *Arabidopsis thaliana*.

The "Amaranthaceae" plants may include, but are not limited to, for example, chard, spinach, sugar beet, and quinoa.

The "Amaryllidaceae" plants may include, but are not limited to, for example, scallion, onion, garlic, chive, leek, and shallot.

The "Apiaceae" plants may include, but are not limited to, for example, anise, caraway, carrot, celery, chervil, coriander, cumin, dill, fennel, parsley, and parsnip.

The "Asparagaceae" plants may include, but are not limited to, for example, agave and asparagus.

The "Asteraceae" plants may include, but are not limited to, for example, artichoke, aster, chamomile, chicory, chrysanthemum, dahlia, daisy, echinacea, goldenrod, guayule, lettuce, marigold, safflower, sunflower, and zinnia.

The "Bromeliaceae" plants may include, but are not limited to, for example, pineapple.

The "Convolvulaceae" plants may include, but are not limited to, for example, morning glory, and sweet potato.

The "Fabaceae" plants may include, but are not limited to, for example, alfalfa, beans, carob, clover, guar, lentils, mesquite, peas, peanuts, soybeans, tamarind, tragacanth, and vetch.

The "Malvaceae" plants may include, but are not limited to, for example, cacao, cotton, durian, hibiscus, kenaf, cola, and okra.

The "Musaceae" plants may include, but are not limited to, for example, banana and plantain.

The "Poaceae" plants may include, but are not limited to, for example, bamboo, barley, maize, fonio, millet, oats, ornamental grasses, rice, rye, sorghum, sugar cane, triticale, wheat, and turfgrasses such as bahiagrass, bermudagrass, bluegrass, buffalograss, centipedegrass, fescue, or zoysia.

The "Polygonaceae" plants may include, but are not limited to, for example, buckwheat.

The "Rosaceae" plants may include, but are not limited to, for example, almond, apple, apricot, blackberry, blueberry, cherry, peach, plum, quince, raspberry, rose, and strawberry.

The "Rubiaceae" plants may include, but are not limited to, for example, coffee.

The "Solanaceae" plants may include, but are not limited to, for example, bell pepper, chili pepper, eggplant, petunia, potato, tobacco, and tomato.

The "Vitaceae" plants may include, but are not limited to, for example, grape.

According to one embodiment, the N474 CP02-1004 strain and/or the N481 CP02-1005 strain may have activities for enhancing plant stress resistance and/or promoting plant growth. Specifically, when cucumbers (*Cucumis sativus*) are grown under drought stress conditions, both the fresh weight and the plant water content of the cucumbers are reduced, but when the cucumbers are treated with the N474 CP02-1004 strain or the N481 CP02-1005 strain, both the fresh weight and the plant water content of the cucumbers reduced by drought stress may be restored.

In one embodiment, the N474 CP02-1004 strain or the N481 CP02-1005 strain may increase the fresh weight of cucumbers reduced by drought stress, by 5 to 50%, 10 to 49%, 15 to 48%, 20 to 47%, or 25 to 45%.

In one embodiment, the N474 CP02-1004 strain or the N481 CP02-1005 strain may increase the plant water content of cucumbers reduced by drought stress, by 5 to 40%, 7 to 38%, 9 to 36%, 10 to 34%, 11 to 32%, 12 to 30%, 13 to 28%, or 14 to 26%, or 15 to 25%.

That is, the N474 CP02-1004 strain or the N481 CP02-1005 strain may enhance plant stress resistance and promote plant growth, and thus may be usefully utilized for the purpose of enhancing plant stress resistance and promoting plant growth.

### 2. Use of the novel strains of the present disclosure

According to another aspect of the present disclosure, there is provided a bacterial composition comprising, as an active ingredient, the *Bacillus amyloliquefaciens* N474 CP02-1004 strain deposited under Accession No. KCCM13393P or the *Bacillus amyloliquefaciens* N481 CP02-1005 strain deposited under Accession No. KCCM13394P.

In one embodiment, the composition may further comprise a cryoprotectant.

The *Bacillus amyloliquefaciens* N474 CP02-1004 strain deposited under Accession No. KCCM13393P or the *Bacillus amyloliquefaciens* N481 CP02-1005 strain deposited under Accession No. KCCM13394P may be prepared in the form of a lyophilized composition to preserve the activity of these microbial cells, and specifically, may be prepared in the form of a lyophilized powder, and the composition may further comprise a cryoprotectant to prevent damage to the cells.

In one embodiment, the cryoprotectant may include, but is not limited to, one or more selected from the group consisting of glycerol, ethylene glycol, propylene glycol, trehalose, sucrose, glucose, maltodextrin, sorbitol, skim milk powder, and starch.

In one embodiment, the cryoprotectant may be included in an amount of 0.01 to 30% by weight based on the total composition, specifically 0.01 to 30% by weight, 0.01 to 25% by weight, 0.01 to 20% by weight, 0.01 to 15% by weight, 0.1 to 30% by weight, 0.1 to 25% by weight, 0.1 to 20% by weight, 0.1 to 15% by weight, 1 to 30% by weight, 1 to 25% by weight, 1 to 20% by weight, or 1 to 15% by weight based on the total composition.

In one embodiment, the N474 CP02-1004 strain or the N481 CP02-1005 strain is included as an active ingredient in the composition, and therefore, the concentration thereof may be 1 x 10⁵ CFU/mL to 1 x 10¹² CFU/mL. For example, the concentration of the strain may be, but is not limited to, 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

Another aspect of the present disclosure provides a composition for enhancing plant stress resistance.

Specifically, the composition for enhancing plant stress resistance of the present disclosure may comprise, as an active ingredient, one or more selected from the group consisting of the *Bacillus amyloliquefaciens* N474 CP02-1004 strain deposited under Accession No. KCCM13393P, a culture of the strain, a concentrate of the culture, an extract of the culture, a dried product of the culture, and a pulverized product of the dried product.

In addition, the composition for enhancing plant stress resistance of the present disclosure may comprise, as an active ingredient, one or more selected from the group consisting of the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

The overlap between the contents of one aspect of the composition for enhancing plant stress resistance and the contents described in "***1. Novel strains and activity thereof***" is applied in the same manner as described above, and therefore, it is cited and is not described repeatedly to avoid excessive complexity of the specification.

In the present disclosure, the term "culture" refers to a product obtained by culturing the strain, and the culture may encompass the culture broth itself in which the strain has been cultured, a culture filtrate obtained by removing the strain through filtration or centrifugation of the culture broth, and a supernatant obtained by centrifuging the culture broth or culture filtrate.

In the present disclosure, the term "concentrate" refers to the culture having an increased solids concentration, and may be a concentrate of the culture containing cells of the strain of the present disclosure, or a concentrate of the culture supernatant from which cells of the strain of the present disclosure has been removed. The concentrate may be, but is not limited to, those concentrated by vacuum concentration, plate-type concentration, thin film concentration, or the like, for example, it may be carried out at a temperature of 40 °C to 60 °C using a known concentrator. The content of the culture included in the composition of the present disclosure may be appropriately adjusted according to the concentration of the concentrate.

In the present disclosure, the term "extract" refers to a product obtained by extracting the culture using an appropriate solvent according to a known method. The extract means an extract from the culture or a concentrate thereof, and may include an extract liquid, a diluent or concentrate of the extraction liquid, a dried product obtained by drying the extract liquid, a crude purified product or purified product thereof, or a fraction obtained by fractionating the same.

The term "dried product" refers to a product obtained by removing moisture from the culture or concentrate, and may be, but is not limited to, for example, a dried cell form of the strain. The dried product includes, but is not limited to, those dried by methods such as freeze drying, vacuum drying, hot air drying, spray drying, reduced pressure drying, spray drying, foam drying, high frequency drying, and infrared drying.

In addition, the term "pulverized product" refers to a product obtained by pulverizing a dried product from which moisture has been removed from the cells or culture of the strain, and may be, but is not limited to, for example, a dried cell powder of the strain.

The culture of the strain may be prepared by inoculating the strain into a culture medium and culturing it according to a culture method known in the art, and the concentrate, extract, dried product or pulverized product may be prepared according to methods for treating or drying the strain or its culture known in the art.

In one embodiment of the composition for enhancing plant stress resistance, the stress may be drought stress.

In one embodiment of the composition for enhancing plant stress resistance, the plant may be one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

According to one embodiment, one or more selected from the group consisting of the *Bacillus amyloliquefaciens* N474 CP02-1004 strain deposited under Accession No. KCCM13393P, a culture of the strain, a concentrate of the culture, an extract of the culture, a dried product of the culture, and a pulverized product of the dried product may effectively mitigate the reduction in fresh weight and the reduction in plant water content of plants caused by drought stress, and thus may be usefully utilized for the purpose of enhancing plant stress resistance.

In addition, according to one embodiment, one or more selected from the group consisting of the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product may effectively mitigate the reduction in fresh weight and the reduction in plant water content of plants caused by drought stress, and thus may be usefully utilized for the purpose of enhancing plant stress resistance.

In one embodiment, the composition may comprise the strain, a culture of the N474 or N481 strain, a concentrate of the culture, an extract of the culture, a dried product of the culture, and a pulverized product of the dried product in an amount of 0.00001% by weight to 80% by weight, for example, 0.00001% by weight to 60% by weight, 0.00001% by weight to 40% by weight, 0.00001% by weight to 30% by weight, 0.00001% by weight to 20% by weight, 0.00001% by weight to 10% by weight, 0.00001% by weight to 5% by weight, 0.05% by weight to 60% by weight, 0.05% by weight to 40% by weight, 0.05% by weight to 30% by weight, 0.05% by weight to 20% by weight, 0.05% by weight to 10% by weight, 0.05% by weight to 5% by weight, 0.1% by weight to 60% by weight, 0.1% by weight to 40% by weight, 0.1% by weight to 30% by weight, 0.1% by weight to 20% by weight, 0.1% by weight to 10% by weight, or 0.1% by weight to 5% by weight, based on the total weight of the composition.

In one embodiment, the concentration of the N474 CP02-1004 strain in the composition for enhancing plant stress resistance may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In addition, in one embodiment, the concentration of the N481 CP02-1005 strain in the composition for enhancing plant stress resistance may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In one embodiment, when the composition for enhancing plant stress resistance is in a solid state rather than a liquid state, the unit "CFU/mL" used to describe the concentration of the strain may be changed to "CFU/g".

In the present disclosure, the composition for enhancing plant stress resistance may be prepared in the form of a directly sprayable solution, in the form of powder and suspension, or as a highly concentrated aqueous, oily or other suspension, dispersion, emulsion, oil dispersion, paste, dust, scatterable material, or granule, but is not limited thereto.

In the present disclosure, the composition for enhancing plant stress resistance may be formulated in various forms. The formulations may be prepared, for example, by adding solvents and/or carriers, and inert additives and surface-active substances, for example, emulsifiers or dispersants, may be mixed into the formulations. Suitable surface-active substances may be, but are not limited to, aromatic sulfonic acids (e.g. lignosulfonic acid, phenolsulfonic acid, naphthalene- and dibutylnaphthalenesulfonic acid), fatty acids, alkyl- and alkylarylsulfonates, alkyl lauryl ethers, alkali metal, alkaline earth metal and ammonium salts of fatty alcohol sulfates, sulfated hexa-, hepta- and octadecanol, salts of fatty alcohol glycol ethers, sulfonated naphthalene and derivatives thereof, condensates of formaldehyde, condensates of naphthalene or naphthalenesulfonic acid, phenol and formaldehyde, polyoxyethyleneoctyl phenol ethers, ethoxylated isooctyl-, octyl- or nonylphenols, alkylphenyl or tributylphenyl polyglycol ethers, alkylarylpolyether alcohols, isotridecyl alcohol, fatty alcohol/ethylene oxide condensates, ethoxylated castor oil, polyoxyethylene alkyl ether or polyoxypropylene, lauryl alcohol polyglycol ether acetate, sorbitol ester, lignin-sulfite waste liquor, or methylcellulose.

The carriers may be, but are not limited to, an agriculturally acceptable carrier, such as mineral earths (e.g., silica, silica gel, silicate, talc, kaolin, limestone, lime, chalk, boll, loess, clay, dolomite, diatomaceous earth, calcium sulfate, magnesium sulfate, magnesium oxide, ground synthetic materials), fertilizers (e.g., ammonium sulfate, ammonium phosphate, ammonium nitrate, urea), vegetable products (e.g., cereal flour, tree bark flour, wood meal, and nut shell flour), or cellulose powder, and the carriers may be used alone or in combination of two or more thereof.

In the present disclosure, the composition for enhancing plant stress resistance may further comprise any other component used in the art to enhance plant stress resistance. For example, it may further comprise any one or more selected from the group consisting of a plant growth regulator, a plant growth stimulant, and a plant nutrient.

The plant growth regulator, plant growth stimulant or plant nutrient may be a chemical or another bacterial strain, and may be a herbicide, bactericide, insecticide, fertilizer, or any combination thereof.

In the present disclosure, the composition for enhancing plant stress resistance may further comprise any one or more selected from the group consisting of other beneficial microorganisms, microbial active substances, organic fertilizers, and chemical fertilizers.

Other beneficial microorganisms may include *Bacillus spp.*, *Azotobacter spp.*, *Trichoderma spp.*, and *Saccharomyces spp.*, which are known to enhance plant stress resistance.

The microbial active substances may include enzyme precursors, microbial metabolites, organic acids, carbohydrates, enzymes, and trace elements. For example, the microbial active substances may include processed enzyme products (such as yeast autolysates), humic materials, seaweed extracts, starch, amino acids, and trace elements such as zinc, iron, copper, manganese, bromine, and molybdenum.

The chemical fertilizers may include various chemicals that can provide nitrogen, phosphorus and potassium nutrients to help enhance plant stress resistance. For example, the chemical fertilizers may include urea, calcium phosphate, potassium phosphate, and mixed nitrogen-phosphate-potassium (N-P-K) fertilizers, and may include other materials known in the art.

In the present disclosure, the composition for enhancing plant stress resistance may further comprise a spreading agent, a penetrating agent, or a surfactant to increase its absorption and effectiveness in plants.

In the present disclosure, the composition for enhancing plant stress resistance may further comprise preservatives, stabilizers, wetting agents, emulsifiers, cryoprotectants, or excipients.

For example, the composition for enhancing plant stress resistance may comprise the preservatives, stabilizers, or excipients in an effective amount sufficient to reduce the quality deterioration of the composition, thereby allowing the N474 CP02-1004 strain and/or the N481 CP02-1005 strain to survive or remain active for a longer period of time in the composition, unlike the N474 CP02-1004 strain and/or the N481 CP02-1005 strain that exist in nature.

The preservatives, stabilizers, or excipients may include, without limitation, those commonly used in the art as long as they can reduce the quality deterioration of the composition for enhancing plant stress resistance. For example, the stabilizers may be any one or more selected from the group consisting of alginate, sodium alginate, casein, sodium caseinate, cellulose, methylcellulose, carboxymethylcellulose, sodium carboxymethylcellulose, pectin, gelatin, gum arabic, xanthan gum, dextran, cyclodextrin, and starch. The excipients may include pharmaceutically acceptable solid carriers, liquid carriers, liquid diluents, liquefied gaseous diluents, solid diluents, or other adjuvants such as surfactants, for example, emulsifiers, dispersants or foaming agents.

The cryoprotectants may reduce or prevent damage to the N474 CP02-1004 strain and/or N481 CP02-1005 strain, which exist in nature, caused by freezing during the freeze-drying process.

The cryoprotectants may be included in the composition in an effective amount sufficient to reduce the quality deterioration of the composition when the composition for enhancing plant stress resistance is in a lyophilized state. For example, unlike the N474 CP02-1004 strain and/or the N481 CP02-1005 strain that exist in nature, the composition may comprise the cryoprotectants in an effective amount sufficient to reduce the quality deterioration of the composition or strain in a lyophilized state, thereby allowing the N474 CP02-1004 strain and/or the N481 CP02-1005 strain to survive or remain active for a longer period of time within the lyophilized composition.

The cryoprotectants may include, without limitation, those commonly used in the art as long as they can reduce the quality deterioration of the composition or strain in a lyophilized state. For example, the cryoprotectants may be any one or more selected from the group consisting of glycerol, ethylene glycol, propylene glycol, dimethyl sulfoxide (DMSO), glucose, trehalose, maltodextrin, dextrin, skim milk powder, and starch. In addition, it is used by mixing with a preservative carrier, adsorbing, and then drying, and solidifying, wherein the preservative carrier may be diatomaceous earth, activated carbon, and/or defatted rice bran.

In one embodiment, the composition for enhancing plant stress resistance may be in the form of a lyophilized powder.

The description of the strains, carriers, excipients and additives is the same as described above. When the cryoprotectants are used as the additives, the novel *Bacillus spp.* strain of the present disclosure may be prepared in the form of a lyophilized powder by mixing the strain and the cryoprotectants, subjecting the mixture to a freezing process at -45 °C to -30 °C, and then drying at 30 °C to 40 °C and grinding with a mixer. Specifically, the freezing process may be a process of vacuum freezing for 65 to 75 hours under a temperature condition of -45°C to -30°C and a pressure condition of 5 to 50 mTorr.

When the composition of the present disclosure further comprises the cryoprotectants, damage or death of the strain may be suppressed during the freeze-drying process of the composition, and the composition in freeze-dried form has advantageous advantages in the storage, distribution, and preservation processes. In addition, the composition in freeze-dried form may be distributed in the form of a powder, in which case the strain in the composition may exhibit its activity while growing or metabolizing in a subject under drought stress.

In the present disclosure, the amount of the composition to be used may be appropriately determined depending on the formulation, damage situation, application method, application location, etc.

In one embodiment, the composition for enhancing plant stress resistance may be added to a plant fertilizer by drenching, spraying, or mixing.

The plant fertilizer may be prepared by a preparation method commonly used in the art. For example, the plant fertilizer may further comprise excipients or carriers commonly used in the art, and may be formulated into conventional plant fertilizer formulations known in the art, such as wettable powders, granules, dusts, emulsifiables, sprays, smokes, capsules, or gels.

In one embodiment, the plant fertilizer may be an environmentally friendly fertilizer capable of replacing chemical fertilizers, and the plant fertilizer may be used to enhance plant stress resistance.

Another aspect of the present disclosure provides a method for enhancing plant stress resistance.

The method for enhancing plant stress resistance of the present disclosure comprises treating a plant, an area surrounding the plant, or both, with one or more selected from the group consisting of the *Bacillus amyloliquefaciens* N474 CP02-1004 strain deposited under Accession No. KCCM13393P, a culture of the strain, a concentrate of the culture, an extract of the culture, a dried product of the culture, and a pulverized product of the dried product.

In addition, the method for enhancing plant stress resistance of the present disclosure comprises treating a plant, an area surrounding the plant, or both, with one or more selected from the group consisting of the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

In one embodiment, the plant stress may be drought stress.

In the present disclosure, the plant may include any of roots, stems, leaves, seeds, fruits, or parts or combinations thereof, without limitation to the part and type of a plant. Specifically, the plant may be, but is not limited to, a plant individual capable of growing under stress conditions or one that has been grown under stress conditions.

In the present disclosure, the term "area surrounding the plant" refers to the range in which the active ingredient of the present disclosure may exert its effect, and includes both above-ground and underground areas. For example, it may be an area within about 2 m, within about 1 m, within about 70 cm, within about 50 cm, within about 25 cm, within about 10 cm, or within about 5 cm around a plant, a part of the plant, or fruit.

According to one embodiment, the method for enhancing plant stress resistance may effectively restore the fresh weight and the plant water content of plants reduced by drought stress, and thus may effectively enhance plant stress resistance.

Treatment of a plant with one or more selected from the group consisting of the strain, the culture of the strain, the concentrate of the culture, the extract of the culture, the dried product of the culture, and the pulverized product of the dried product may be performed by methods such as dipping, drenching, spraying, injection, infusion, mixing, or soil incorporation, but is not limited thereto as long as they can come into direct or indirect contact with the inside or outside of the plant. In addition, the treatment may be performed simultaneously with or separately from the growth of the plant under stress conditions, but is not limited thereto. For example, the treatment may be performed once or two or more times, but is not limited thereto. In addition, in the case of two or more treatments, the treatments may be performed at intervals of 3 to 15 days, 3 to 10 days, 3 to 7 days, 4 to 7 days, 5 to 7 days, or 6 days, but are not limited thereto.

Another aspect of the present disclosure provides the use of a composition comprising one or more selected from the group consisting of the *Bacillus amyloliquefaciens* N474 CP02-1004 strain deposited under Accession No. KCCM13393P, a culture of the strain, a concentrate of the culture, an extract of the culture, a dried product of the culture, and a pulverized product of the dried product in enhancing plant stress resistance.

In one embodiment, the plant stress may be drought stress.

In addition, another aspect of the present disclosure provides the use of a composition comprising one or more selected from the group consisting of the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product for enhancing plant stress resistance.

In one embodiment, the plant stress may be drought stress.

Another aspect of the present disclosure provides a composition for promoting plant growth.

The composition for promoting plant growth of the present disclosure comprises, as an active ingredient, one or more selected from the group consisting of the *Bacillus amyloliquefaciens* N474 CP02-1004 strain deposited under Accession No. KCCM13393P, a culture of the strain, a concentrate of the culture, an extract of the culture, a dried product of the culture, and a pulverized product of the dried product.

In addition, the composition for promoting plant growth of the present disclosure comprises, as an active ingredient, one or more selected from the group consisting of the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

The overlap between the contents of one aspect of the composition for promoting plant growth and the contents described in the aforementioned "***1. Novel strains and activity thereof***" and "***2. Use of novel strains of the present disclosure***" is applied in the same manner as described above, and therefore, it is cited and is not described repeatedly to avoid excessive complexity of the specification.

In one embodiment of the composition for promoting plant growth, the plant may be one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

According to one embodiment, one or more selected from the group consisting of the *Bacillus amyloliquefaciens* N474 CP02-1004 strain deposited under Accession No. KCCM13393P, a culture of the strain, a concentrate of the culture, an extract of the culture, a dried product of the culture, and a pulverized product of the dried product may effectively increase the fresh weight and the plant water content of plants reduced by drought stress, and thus may be usefully utilized for the purpose of promoting plant growth.

In addition, according to one embodiment, one or more selected from the group consisting of the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product may effectively increase the fresh weight and the plant water content of plants reduced by drought stress, and thus may be usefully utilized for the purpose of promoting plant growth.

In one embodiment, the concentration of the N474 CP02-1004 strain in the composition for promoting plant growth may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In addition, in one embodiment, the concentration of the N481 CP02-1005 strain in the composition for promoting plant growth may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In one embodiment, when the composition for promoting plant growth is in a solid state rather than a liquid state, the unit "CFU/mL" used to describe the concentration of the strain may be changed to "CFU/g".

In the present disclosure, the composition for promoting plant growth may be prepared in the form of a directly sprayable solution, in the form of powder and suspension, or as a highly concentrated aqueous, oily or other suspension, dispersion, emulsion, oil dispersion, paste, dust, scatterable material, or granule, but is not limited thereto, and may be formulated into various forms.

In the present disclosure, the composition for promoting plant growth may further comprise any other component used in the art to enhance plant stress resistance. For example, it may further comprise any one or more selected from the group consisting of a plant growth regulator, a plant growth stimulant, and a plant nutrient.

In the present disclosure, the composition for promoting plant growth may further comprise any one or more selected from the group consisting of other beneficial microorganisms, microbial active substances, organic fertilizers, and chemical fertilizers.

In the present disclosure, the composition for promoting plant growth may further comprise a spreading agent, a penetrating agent, or a surfactant to increase its absorption and effectiveness in plants.

In the present disclosure, the composition for promoting plant growth may further comprise preservatives, stabilizers, wetting agents, emulsifiers, cryoprotectants, or excipients.

In one embodiment, the composition for promoting plant growth may be added to a plant fertilizer by drenching, spraying, or mixing.

In one embodiment, the plant fertilizer may be an environmentally friendly fertilizer capable of replacing chemical fertilizers, and the plant fertilizer may be used to promote plant growth.

Another aspect of the present disclosure provides a method for promoting plant growth.

The method for promoting plant growth of the present disclosure comprises treating a plant, an area surrounding the plant, or both, with one or more selected from the group consisting of the *Bacillus amyloliquefaciens* N474 CP02-1004 strain deposited under Accession No. KCCM13393P, a culture of the strain, a concentrate of the culture, an extract of the culture, a dried product of the culture, and a pulverized product of the dried product.

In addition, the method for promoting plant growth of the present disclosure comprises treating a plant, an area surrounding the plant, or both, with one or more selected from the group consisting of the *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

In the present disclosure, the plant may include any of roots, stems, leaves, seeds, fruits, or parts or combinations thereof, without limitation to the part and type of a plant. Specifically, the plant may be, but is not limited to, a plant individual in need of growth promotion. According to one embodiment, the method for promoting plant growth may effectively increase the reduced fresh weight and plant water content of plants, and thus may effectively promote plant growth.

In one embodiment, the reduction in the fresh weight of the plants and the reduction in the water content within the plants may be induced by drought stress.

In one embodiment, the promoting plant growth may be promoting the plant growth that has been reduced by drought stress.

Treatment of a plant with one or more selected from the group consisting of the strain, the culture of the strain, the concentrate of the culture, the extract of the culture, the dried product of the culture, and the pulverized product of the dried product may be performed by methods such as dipping, drenching, spraying, injection, infusion, mixing, or soil incorporation, but is not limited thereto as long as they can come into direct or indirect contact with the inside or outside of the plant. For example, the treatment may be performed once or two or more times, but is not limited thereto. In addition, in the case of two or more treatments, the treatments may be performed at intervals of 3 to 15 days, 3 to 10 days, 3 to 7 days, 4 to 7 days, 5 to 7 days, or 6 days, but are not limited thereto.

Another aspect of the present disclosure provides the use of a composition comprising one or more selected from the group consisting of the N474 CP02-1004 strain, a culture of the strain, a concentrate of the culture, an extract of the culture, a dried product of the culture, and a pulverized product of the dried product in promoting plant growth.

In addition, another aspect of the present disclosure provides the use of a composition comprising one or more selected from the group consisting of the N481 CP02-1005 strain, a culture of the strain, a concentrate of the culture, an extract of the culture, a dried product of the culture, and a pulverized product of the dried product in promoting plant growth.

### Advantageous Effects

The *Bacillus amyloliquefaciens* N474 CP02-1004 strain, the *Bacillus amyloliquefaciens* N481 CP02-1005 strain, or a culture thereof of the present disclosure may effectively increase the fresh weight and plant water content of plants reduced by drought stress. Therefore, the strain or a culture thereof of the present disclosure can be usefully utilized as an environmentally friendly product that can promote plant production by enhancing drought stress resistance in plants.

However, the effects of the present disclosure are not limited to the effects mentioned above, and other effects that are not mentioned will be clearly understood by a person skilled in the art from the description below.

### Description of Drawings

FIG. 1 shows the results of measuring the fresh weight of cucumbers after treating them with a culture of *Bacillus amyloliquefaciens* strain under drought stress conditions.
FIG. 2 shows the results of comparing (A) the fresh weight and (B) RWC (Relative water content) of cucumbers after treating them with a culture of *Bacillus amyloliquefaciens* N474 CP02-1004 strain or a culture of *Bacillus amyloliquefaciens* N481 CP02-1005 strain under drought stress conditions.

### Best Mode

Hereinafter, the present disclosure will be described in detail by examples. However, the following examples are only for specifically illustrating the present disclosure, and the content of the present disclosure is not limited by the following examples.

### Example 1: Isolation and culture of Bacillus amyloliquefaciens strains

The strains used in this experiment were obtained from field soil (farmland located in Yeongtong-gu, Suwon-si, Gyeonggi-do). Approximately 50 g of soil was collected at a depth of 5-10 cm from the ground surface, and then the collected soil was passed through a 2 mm mesh sieve to remove large stones and impurities. Each 5 g of the filtered soil sample was cultured in flasks, and the resulting culture was diluted 1/10 with sterilized distilled water to purely isolate the microorganisms.

Among the isolated microorganisms, six strains showing high antagonistic activity against the representative plant pathogens *Rhizoctonia solani* (R.s), *Pythium ultimum* (P.u), and *Colletotrichum truncatum* (C.t) were selected. To confirm the bactericidal effects against plant pathogens, four paper discs were placed on 8.5cm diameter Petri dishes containing PDA (potato dextrose agarose) medium, and three of the paper discs were inoculated with the reference strain *Bacillus amyloliquefaciens* KACC 10116 (*B. amyloliquefaciens* KACC 10116) strain, Serenade^{®} (Bayer, *B. velezensis* QST713), and the strain to be evaluated. The remaining one paper disc was used as an untreated control. The three plant pathogens (R.s, P.u, C.t) were cultured on PDA (potato dextrose agarose) medium at 28 °C for 5 to 8 days, and then the pathogen colonies were placed in the center of the Petri dishes using a cork borer with a diameter of 5 mm. After culturing in an incubator at 25 °C for 7 to 20 days, the diameter of the zone of inhibition was examined to compare the antibacterial effects of the *Bacillus* strains. Finally, six strains (N311, N474, N481, N484, N485, and N490) showing antibacterial effects equal to or greater than those of Serenade^{®} were selected (Table 1).

**[Table 1]**

| Antibacterial effect (unit: mm) | | | |
|---|---|---|---|
| | *Rhizoctonia solani* | *Phythium ultium* | *Colletotrichum trucatum* |
| N311 | 9.7 | 3.7 | 12.7 |
| N474 | 10.3 | 3.3 | 11 |
| N481 | 9.3 | 5.0 | 12.3 |
| N484 | 8.7 | 1.0 | 11.7 |
| N485 | 4.3 | 4.7 | 6.7 |
| N490 | 9.7 | 4.0 | 10.7 |
| Serenade | 9.7 | 4.0 | 11.7 |

Specifically, the identification of the six selected strains was performed by analyzing the identity of the 16S rRNA gene nucleotide sequence with the previously reported *Bacillus amyloliquefaciens* reference strain KACC 10116 using the CLC Genomics Workbench 21 program (Qiagen). As a result of the analysis, each of the six strains was confirmed to have 99% sequence homology, and they were finally identified as *Bacillus amyloliquefaciens* strains: The following *Bacillus amyloliquefaciens* strains were used: *Bacillus amyloliquefaciens* N474 CP02-1004 strain (Accession No. KCCM13393P), *Bacillus amyloliquefaciens* N481 CP02-1005 strain (Accession No. KCCM13394P), *Bacillus amyloliquefaciens* N484 CP02-1006 strain (Accession No. KCCM13395P), *Bacillus amyloliquefaciens* N485 CP02-1007 strain (Accession No. KCCM13396P), *Bacillus amyloliquefaciens* N490, *Bacillus amyloliquefaciens* N311. Each identified strain was suspended in 20% glycerol and stored.

Among the six finally selected strains, the N474 strain and the N481 strain were found to have the most excellent efficacy in mitigating drought stress according to subsequent experimental results.

The N474 strain was designated *Bacillus amyloliquefaciens* N474 CP02-1004 strain, was deposited internationally with the Korean Culture Center of Microorganisms, which is an international depository institution under the Budapest Treaty, and was assigned the Accession No. KCCM13393P on September 21, 2023. In addition, the N481 strain was designated *Bacillus amyloliquefaciens* N481 CP02-1005 strain, was deposited internationally with the Korean Culture Center of Microorganisms, which is an international depository institution under the Budapest Treaty, and was assigned the Accession No. KCCM13394P on September 21, 2023.

The 16s rRNA gene nucleotide sequences of the N474 strain and the N481 strain are set forth in SEQ ID NO: 1 and SEQ ID NO: 2, respectively.

The six *Bacillus amyloliquefaciens* strains selected and identified as described above were cultured along with a *Bacillus amyloliquefaciens* strain isolated from Serenade (Bayer) as a control. A singe colony of each *Bacillus amyloliquefaciens* strain was streaked onto LB (Luria-Bertani broth) medium and then primarily cultured in an incubator at 28 °C for 20 hours. A colony was picked from the primary culture medium, streaked onto LB medium for a second time, and then secondarily cultured in an incubator at 28 °C for 20 hours. Subsequently, 10 mL of sterilized distilled water was added to the secondary culture medium to perform cell resuspension, and the cells were recovered using a spreader. The O.D (optical density) value of each strain was measured and then diluted to O.D = 1.0 for use in the experiment.

### Example 2: Selection of Bacillus strains with excellent efficacy in mitigating drought stress

An experiment was conducted to confirm the effect of the six *Bacillus amyloliquefaciens* strains cultured in Example 1 above on mitigating plant drought stress.

### 2-1. Experimental method

Cucumber (*Cucumis sativus*) was used as the plant, and cucumber seeds were sown in a 7:3 mixture of horticultural potting soil and vermiculite. Normal cucumber growth conditions were set as temperature 25 °C, humidity 60 to 65%, normal irrigation cycle, and photoperiod 16h/8h (day/night). Drought stress growth conditions were set as water withholding for 14 days, temperature 25 °C, humidity 60-65%, and photoperiod 16h/8h (day/night). Control groups were a DW-treated group under normal conditions, a DW-treated group under drought stress conditions, and a commercial product control under drought stress conditions, wherein the commercial product control was set as a group treated with a culture of the *Bacillus amyloliquefaciens* strain isolated from Serenade (Bayer) (hereinafter referred to as the "Serenade-treated group"). Test groups were set as groups treated with cultures of the six *Bacillus amyloliquefaciens* strains (N474, N481, N484, N485, N490, and N311) cultured in Example 1 above, under drought stress conditions.

Ten days after sowing cucumbers, the cultures (OD = 1.0, 1×10⁸ CFU/mL) of the *Bacillus amyloliquefaciens* strains cultured to an O.D = 1.0 value were foliar-applied in an amount of 5 mL/plant, twice at 7-day intervals. After the first application, drought stress was caused by water withholding. After the cucumbers were grown for 7 days under drought stress conditions, the fresh weight was determined by measuring the total weight of the above-ground part.

### 2-2. Measurement results of fresh weights

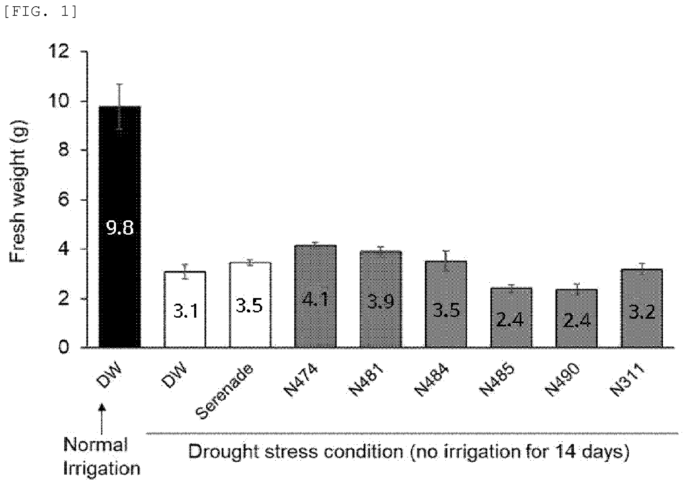

As a result of measuring fresh weight, when drought stress was induced, the fresh weight of cucumbers was remarkably reduced compared to normal conditions, but the fresh weight reduced by drought stress was found to be restored in the *Bacillus amyloliquefaciens* N474 strain-treated group and the N481 strain-treated group (FIG. 1).

Specifically, it was confirmed that based on the final fresh weight, the N474 strain-treated group and the N481 strain-treated group increased fresh weight by approximately 32.2% and 25.8%, respectively, compared to the DW-treated group under drought stress conditions. In addition, it was confirmed that the N474 strain-treated group and the N481 strain-treated group also increased fresh weight by approximately 17.1% and 11.4%, respectively, compared to the Serenade-treated group. That is, it can be seen that the Serenade (i.e., commercial product)-treated group increased the fresh weight by approximately 12.9% compared to the DW-treated group under drought stress conditions, whereas the N474 strain-treated group and the N481 strain-treated group increased fresh weight by approximately 32.2% and 25.8%, respectively, compared to the DW-treated group under drought stress conditions, showing an effect more than twice as good as commercial products in recovering the reduction in fresh weight caused by drought stress. In addition, the groups treated with the N484, N485, N490, and N331 strains showed fresh weights that were lower than or equal to those of the untreated group or the Serenade-treated group.

Accordingly, additional experiments were conducted to confirm whether the N474 strain and the N481 strain exhibit efficacy in mitigating drought stress.

### Example 3: Verification of strains with excellent efficacy in mitigating drought stress

The efficacy of the N474 and N481 strains selected in Example 2 above in alleviating drought stress was verified.

### 3-1. Experimental method

Other conditions were the same as in Example 2-1, but to maximize the biological treatment effect, the cucumbers were drenched with the Bacillus culture at a concentration of 30 mL/pot. As in Example 2, after the first application, drought stress was induced by starting water withholding, and after the induction of drought stress, the Bacillus culture was applied via drenching once, and then, the fresh weight of the cucumbers was measured, and the relative water content (RWC), which is an indicator that best represents the degree of drought stress, was also measured.

### 3-2. Measurement results of fresh weights

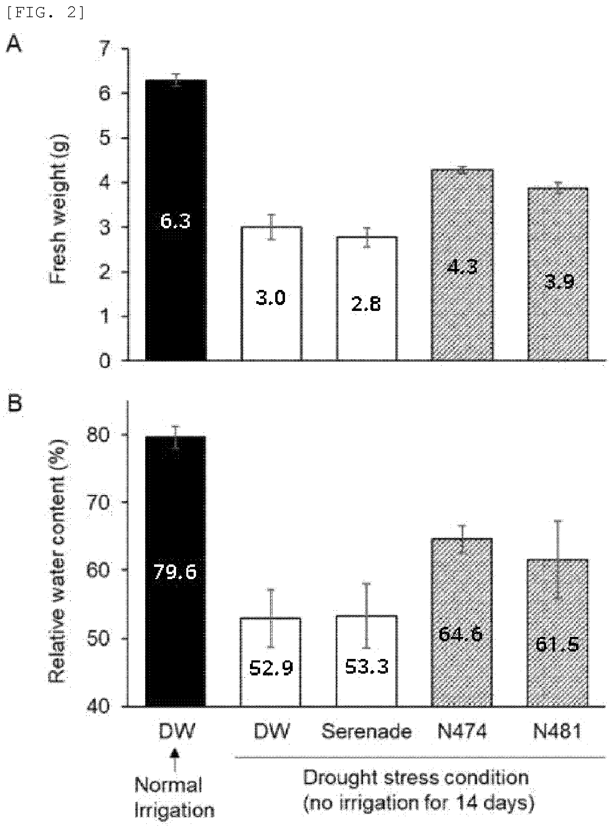

As a result of measuring fresh weight, when drought stress was induced, the fresh weight of cucumbers was remarkably reduced compared to normal conditions, but the fresh weight reduced by drought stress was found to be restored in the N474 strain-treated group and the N481 strain-treated group (Panel A of FIG. 2).

Specifically, it was confirmed that based on the final fresh weight, the N474 strain-treated group and the N481 strain-treated group increased fresh weight by approximately 43.3% and 30%, respectively, compared to the DW-treated group under drought stress conditions. In addition, it was confirmed that the N474 strain-treated group and the N481 strain-treated group also increased fresh weight by approximately 53.5% and 39.2%, respectively, compared to the Serenade-treated group. In the drenching treatment, it was confirmed that the N474 strain-treated group and the N481 strain-treated group were more excellent than the Serenade-treated group in restoring the fresh weight reduced by drought stress.

### 3-3. Measurement results of RWC (relative water content)

RWC is an indicator of the water content in a plant, and normally growing plants exhibit an RWC of approximately 80%. A low RWC indicates a low water content in a plant, whereas a high RWC indicates a high water content in the plant. RWC values were calculated by measuring fresh weight (FW), turgid weight (TW, defined as the weight of plants with saturated water content), and dry weight (DW).

First, the fresh weight (FW) of cucumber plants was measured, and then the plants were submerged in water for 24 hours to saturate the water content in the plant tissue to 100%, and Turgid weight (TW, defined as the weight of plants with saturated water content) was measured. After the measurement of TW, the sample was dried in an oven at 70 °C for approximately 48 hours, and then the dry weight (DW) was measured. Using the FW, TW, and DW values measured in this way, RWC was calculated by the following equation:
$RWC \left(%\right) = \left[\left(FW-{DW}_{¯}\right)/\left(TW-{DW}_{¯}\right)\right] \times 100$

As a result of measuring RWC, when drought stress was induced, the RWC value of cucumbers was remarkably reduced compared to normal conditions, but the RWC reduced by drought stress was found to be restored in the N474 strain-treated group and the N481 strain-treated group (Panel B of FIG. 2).

Specifically, it was confirmed that the RWC values of the N474 strain-treated group and the N481 strain-treated group increased by approximately 22.1% and 16.4%, respectively, compared to the DW-treated group under drought stress conditions. In addition, it was confirmed that the N474 strain-treated group and the N481 strain-treated group also increased plant water content by approximately 21.2% and 15.5%, respectively, compared to the Serenade-treated group. From the above, it was confirmed that the N474 strain-treated group and the N481 strain-treated group were more excellent than the Serenade-treated group in restoring the plant water content reduced by drought stress.

Through the above experiments, it was confirmed that the *Bacillus amyloliquefaciens* N474 strain and N48 strain could effectively mitigate drought stress by preventing water loss within the plant and using water efficiently when plants were subjected to drought stress. Therefore, it is expected that these strains can be usefully utilized for the purpose of enhancing drought stress resistance in plants and promoting plant growth.

Although representative embodiments of the present disclosure have been described above for illustrative purposes, the scope of the present disclosure is not limited to the specific embodiments described above, and appropriate changes will be able to be made by a person skilled in the art within the scope described in the claims of the present disclosure.

[Accession No.]
   Name of depository institution: Korean Culture Center of Microorganisms
Accession No.: KCCM13393P
Accession date: 20230921
Name of depository institution: Korean Culture Center of Microorganisms
Accession No.: KCCM13394P
Accession date: 20230921

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Bacillus amyloliquefaciens N474 CP02-1004 | Accession number given by the
INTERNATIONAL DEPOSITARY AUTHORITY:
KCCM13393P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 21. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 21. 2023

¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Bacillus amyloliquefaciens N481 CP02-1005 | Accession number given by the
INTERNATIONAL DEPOSITARY AUTHORITY:
KCCM13394P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 21. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 21. 2023

¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. A *Bacillus amyloliquefaciens* N474 CP02-1004 strain deposited under Accession No. KCCM13393P, or a *Bacillus amyloliquefaciens* N481 CP02-1005 strain deposited under Accession No. KCCM13394P.

2. The strain according to claim 1, wherein the strain enhances plant stress resistance.

3. The strain according to claim 2, wherein the stress is drought stress.

4. The strain according to claim 1, wherein the strain promotes plant growth.

5. A composition for enhancing plant stress resistance, comprising, as an active ingredient, one or more selected from the group consisting of a *Bacillus amyloliquefaciens* N474 CP02-1004 strain deposited under Accession No. KCCM13393P or a *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

6. The composition for enhancing plant stress resistance according to claim 5, wherein the stress is drought stress.

7. The composition for enhancing plant stress resistance according to claim 5, wherein the plant is one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

8. A composition for promoting plant growth, comprising one or more selected from the group consisting of a *Bacillus amyloliquefaciens* N474 CP02-1004 strain deposited under Accession No. KCCM13393P or a *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

9. The composition for promoting plant growth according to claim 8, wherein the plant is one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

10. A method for enhancing plant stress resistance, comprising treating a plant, an area surrounding the plant, or both, with one or more selected from the group consisting of a *Bacillus amyloliquefaciens* N474 CP02-1004 strain deposited under Accession No. KCCM13393P or a *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.

11. A method for promoting plant growth, comprising treating a plant, an area surrounding the plant, or both, with one or more selected from the group consisting of a *Bacillus amyloliquefaciens* N474 CP02-1004 strain deposited under Accession No. KCCM13393P or a *Bacillus amyloliquefaciens* N481 CP02-1005 strain deposited under Accession No. KCCM13394P, a culture of the strain, a concentrate of the culture, an extract of the culture, a dried product of the culture, and a pulverized product of the dried product.
